# EUROPÄISCHE PATENTSCHRIFT

(11) **EP 1 161 230 B1**
(45) Veröffentlichungstag und Bekanntmachung des Hinweises auf die Patenterteilung: **29.10.2003**
(21) Anmeldenummer: 00916912.9
(22) Anmeldetag: 09.03.2000
(51) Int. Cl.: A61K 9/70

(54) **TRANSDERMALES THERAPEUTISCHES SYSTEM UND VERFAHREN ZU SEINER HERSTELLUNG**
TRANSDERMAL THERAPY SYSTEM AND METHOD FOR PRODUCING THE SAME
SYSTEME TRANSDERMIQUE THERAPEUTIQUE ET SON PROCEDE DE PRODUCTION

(30) Priorität: 18.03.1999 US 124957 P; 19.03.1999 DE 19912477
(43) Veröffentlichungstag der Anmeldung: 12.12.2001
(73) Patentinhaber: LTS Lohmann Therapie-Systeme AG, 56626 Andernach (DE)
(72) Erfinder: KLEIN, Robert-Peter, D-56567 Neuwied (DE); MECONI, Reinhold, D-56567 Neuwied (DE)
(74) Vertreter: Schmidt, Werner, Dr.
(86) Internationale Anmeldenummer: EP0002042
(87) Internationale Veröffentlichungsnummer: WO00056290

(56) Entgegenhaltungen:
- EP-A- 0 306 636
- WO-A-94/06419
- WO-A-97/35564
- GB-A- 845 841
- US-A- 4 879 119
- DATABASE WPI Section Ch, Week 198108 Derwent Publications Ltd., London, GB; Class A96, AN 1981-12555D XP002142198 & JP 55 160717 A (TOYO INK MFG CO), 13. Dezember 1980 (1980-12-13)

## Beschreibung

Therapeutische Systeme zur transdermalen Verabreichung von Arzneimitteln, wie Nikotin, Nitroglycerin, Sexualhormonen, Scopolamin, Fentanyl sind bekannt. Geeignete Systeme sind z.B. in der Internationalen Anmeldung WO 88/01516 beschrieben. Derartige Systeme enthalten als wesentliche Merkmale eine der Haut abgewandte und für den Wirkstoff undurchlässige Rückschicht, mindestens ein Wirkstoffdepot, eine Wirkstoffverteilungseinrichtung, die mit dem Wirkstoffdepot in Verbindung steht, eine Steuereinrichtung, die die Abgabe des Wirkstoffs durch das System steuert, und eine haftklebende Fixierungseinrichtung für das therapeutische System auf der Haut. Dabei kann die Wirkstoffverteilungseinrichtung mit der Steuereinrichtung zu einer Reservoirmatrix kombiniert sein, die eine oder mehrere räumlich definiert zueinander angeordnete, diskrete Wirkstoffdepots mit einer höheren Wirkstoffkonzentration als in der Reservoirmatrix aufweist.

In der WO 88/01516 ist angegeben, daß das Depot auch inerte Hilfsstoffe aufweisen kann wie Stützmaterialien, welche das Wirkstoffdepot gegenüber Druck- und Zug-Anwendung unempfindlich machen, sowie Trägerstoffe. Das Stützmaterial kann nach der US-PS 5,820,876 als inerter Hilfsstoff ein planares Gewebe (Stützgewebe) sein, durch das die Verteilung des Wirkstoffs innerhalb des Depots bewirkt und gefördert wird. Eine spezielle Ausführungsform ist auch in der Figur 5 beider Druckschriften offenbart, wonach sich auf einer Rückschicht eine Klebstoffschicht befindet, auf der der Wirkstoff, ggf. mit Hilfsstoffen, wie Material zum Erleichtern der Verarbeitbarkeit des Wirkstoffs, oder Trägermaterialien, wie Geweben versehen ist. Das Stützgewebe kann auch als Vlies vorliegen. Als geeignet sind in den Beispielen Vliesstoffe (Fasergemisch Zellwolle/Baumwolle 50:50 mit einem Flächengewicht von 80 g/m², Paratex II/80 der Firma Lohmann GmbH & Co. KG bzw. Fasergemisch Zellwolle/Baumwolle 70:30 mit einem Flächengewicht von 40 g/m², Paratex III/40 der Firma Lohmann GmbH & Co. KG) offenbart. In beiden Beispielen heißt es erläuternd, daß der Vliesstoff als Stützgewebe bzw. zur Unterstützung der gleichmäßigen Verteilung des Nikotins als inerter Hilfsstoff im Sinne der Beschreibungseinleitung wirkt.

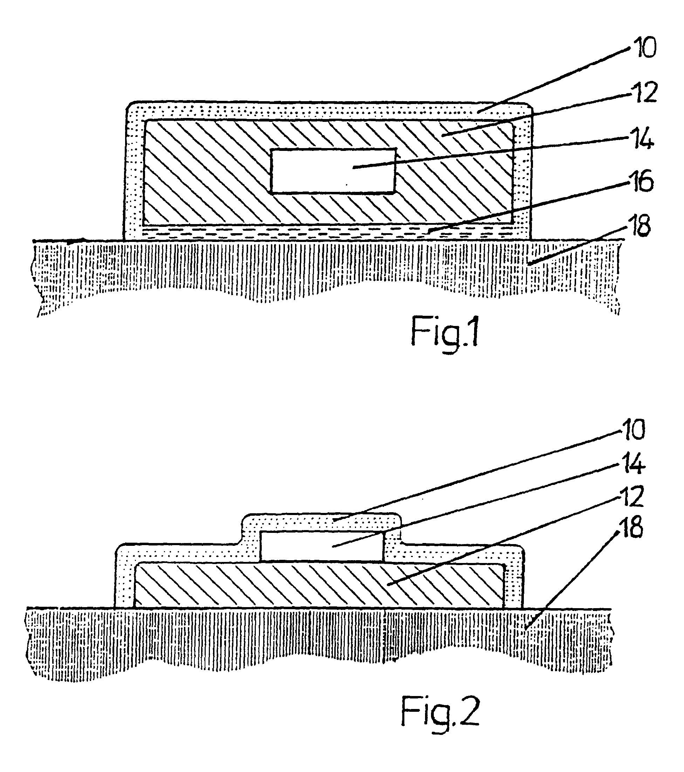

Eine andere Form eines transdermalen therapeutischen Systems wird in der US-PS 4,597,961 beschrieben. Danach wird die Abgabe des Wirkstoffs in der Regel durch eine mikroporöse Membran gesteuert. Bei der Beschreibung der Figur 2 wird erwähnt, daß das Reservoir 114 ein geeignetes absorbierendes Material 122 enthalte, wie einen Schwamm oder Baumwolle, auf dem die gewünschte Menge an flüssigem Nikotin absorbiert sei. Ergänzend wird im Beispiel 4 ausgeführt, das Reservoir 114 enthalte eine dichte Matrix eines inerten faserigen oder porösen Materials, wie Baumwolle, um einen Verlust von Nikotin zu vermeiden. Der Begriff "Matrix" wird hier jedoch für einen gänzlich anderen technischen Gegenstand gebraucht als in der WO 88/01516 und der US-PS 5,820,876.

Bekannt ist ferner aus der US-PS 4,915,950 ein TTS für Nikotin, bei dem sich eine Depotschicht (13) zwischen einer als Steuereinrichtung wirkenden Klebstoffschicht (14) und einem Verankerungskleber (12) befindet. Die Wirkstoffdepotschicht kann aus Vlies, z. B. Polyester, Polyethylen, Polypropylen, Polyamiden, Viskose (Rayon) oder Baumwolle und insbesondere aus 100%-igem Polyestervlies bestehen. Die Verwendung von Papier wird hierin weder beschrieben noch dadurch nahegelegt.

Es wurde nun gefunden, daß man TTS mit gegenüber dem bekannten Stand der Technik erheblich verbesserter Qualität erhält, wenn man an Stelle der bekannten Stützmaterialien, insbesondere auch Geweben wie Vlies, als Trägerstoff Papier verwendet. Papier unterscheidet sich grundsätzlich von Geweben einschließlich Vliesstoffen dadurch, daß in ihm die Cellulosefasern zu einer dünnen Schicht durch Verfestigung verbunden sind. Im Papier beruht der Zusammenhalt der Fasern, abgesehen von der mechanischen Haftung und dem Verhaken der Fasern, auf chemischen Bindungen (Wasserstoffbrücken), die sich bei der Papierherstellung zwischen den Hydroxylgruppen der Cellulosemoleküle ausbilden. Diese chemische Bindung ist so stark, daß Papier in der Zugfestigkeit sogar gewöhnlichen Baustahl übertreffen kann (RM Consult Papiermaschinen Info - http: //home.tonline.de/home/rm.consult/rm-info.htm vom 17.11.1998). Papier hat darüber hinaus den Vorteil, daß es ein hohes Flüssigphasenaufnahmevermögen besitzt, das sich nach DIN ISO 8787 durch die Saughöhe kennzeichnen läßt. So wurde für Papier mit einem Flächengewicht von 26 g/m² eine Saughöhe in Längsrichtung von 146 mm/10min und in Querrichtung von 143 mm/10min gemessen gegenüber Werten von ca. 110 und 80 mm/10min bei dem obengenannten Vliesstoff Paratex III/40, wobei im Reihenversuch die Werte für den Vliesstoff sehr stark streuten. Papier enthält in der Regel kein Bindemittel, so daß Unverträglichkeiten zwischen Wirkstoff und Bindemittel nicht auftreten können.

Gegenstand der Erfindung ist also ein transdermales therapeutisches System, enthaltend als wesentliche Merkmale
a) eine der Haut abgewandte, für den Wirkstoff undurchlässige Rückschicht,
b) mindestens ein Wirkstoffdepot,
c) eine Matrix, die mit dem Wirkstoffdepot in Verbindung steht und die Abgabe des Wirkstoffs steuert, und
d) eine haftklebende Fixierungseinrichtung für das therapeutische System auf der Haut,
wobei das Depot und/oder die Matrix noch Stützmaterialien enthalten, das dadurch gekennzeichnet ist, daß das Stützmaterial aus Papier besteht.

Die erfindungsgemäße Verwendung von Papier als Stützmaterial und inertern Hilfsstoff hat mehrere Vorteile. Bei der Verwendung von Geweben, wie Vliesen, tritt trotz guter Dosiertechnik immer noch eine gewisse Streuung der auf das einzelne TTS übertragenen Wirkstoffmenge auf. Beispielsweise wurde festgestellt, daß die auf die einzelnen TTS verbrachten Nikotinmengen bei Verwendung eines Vlieses (Fasergemisch Zellwolle/Baumwolle 70:30, Flächengewicht 40 g/m²) eine Streuung von etwa 4 % aufweisen. Verwendet man stattdessen erfindungsgemäß Papier, ist die Streuung erheblich geringer; sie liegt dann je nach Flächengewicht des Papieres deutlich unter 2 %, z. B. bei einem Papier mit einem Flächengewicht von 23 g/m² unter 1,9 % und bei einem Papier mit einem Flächengewicht von 26 g/m² sogar unter 1,2 %. Bevorzugt sind Papiere mit einem Flächengewicht von 9 bis 60, vorzugsweise 15 bis 40 und insbesondere 20 bis 35 g/m².

Die erfindungsgemäße Verwendung von Papier als Stützmaterial in TTS hat jedoch nicht nur für die Gleichförmigkeit der hergestellten TTS Bedeutung, sondern auch für die verfahrenstechnische Herstellung. Nach einem bekannten Verfahren wird eine definierte Menge des Wirkstoffs mit einem Tampon auf das Stützmaterial übertragen. Es liegt in der Natur der Sache, daß dabei eine gewisse Menge des Stützmaterials vom Tampon abgerieben und bei dem Ablösen des Tampons vom Stützmaterial mitgerissen wird. Dies macht es erforderlich, daß der Tampon in gewissen Abständen gereinigt und damit das Herstellungsverfahren unterbrochen werden muß. Bei der erfindungsgemäßen Verwendung von Papier ist der Abrieb deutlich geringer, was dadurch erklärt werden kann, daß die Papierfasern miteinander fester verbunden sind als beispielsweise die Fasern in einem Vlies oder anderem Gewebe. Bekanntlich gibt jedes Gewebe faserige Anteile ab. Durch die erfindungsgemäße Verwendung von Papier wird es nun ermöglicht, daß die Funktionsfähigkeit des Tampons mindestens um das 10-fache, in der Regel sogar um das 50-100-fache verlängert wird, so daß dessen Reinigung und damit eine Unterbrechung des Herstellungsprozesses weit seltener notwendig werden.

Erfindungsgemäße TTS können in verschiedener Weise ausgebildet sein. Geeignete Ausgestaltungen sind in den anliegenden Figuren 1 und 2 wiedergegeben, wobei auch weitere Ausführungsformen möglich sind, z. B. wie sie in der Internationalen Anmeldung WO 88/01516 wiedergegeben sind. Nach den Figuren 1 und 2 bestehen die TTS aus einer Rückschicht (10), einer Reservoirmatrix (12), einem oder mehreren Depots (14) und einer Fixierungseinrichtung (16), die noch mit einer Schutzfolie versehen ist, die vor der Verabreichung abgezogen wird, so daß das System dann auf die Haut (18) angebracht wird. Die Schutzfolie soll naturgemäß ebenfalls für den Wirkstoff undurchlässig sein.

Für die Rückschicht, die Reservoirmatrix, die Fixierungseinrichtung und die Schutzfolien werden dem Fachmann bekannte Materialien verwendet.

Gegenstand der Erfindung ist ferner ein Verfahren zur verbesserten Herstellung von transdermalen therapeutischen Systemen mit verminderter Streuung der aufgetragenen Wirkstoffmengen, das dadurch gekennzeichnet ist, daß der Wirkstoff in üblicher Weise mittels eines Tampons auf ein Stützmaterial aufgetragen wird, das aus Papier besteht. Nach einer bevorzugten Ausführungsform wird durch die erfindungsgemäße Arbeitsweise eine Streuung (relative Standardabweichung) der aufgetragenen Wirkstoffmenge von weniger als 2 %, insbesondere unter 1,2 % erreicht.

Gegenstand der Erfindung ist schließlich auch die Verwendung von Papier als Stütz- und Verteilungsmedium in transdermalen therapeutischen Systemen.

Die erfindungsgemäßen Systeme eignen sich grundsätzlich für alle der transdermalen Verabreichung zugänglichen Wirkstoffe. Insbesondere seien außer den oben erwähnten noch genannt Lidocain, Diphenylhydraminhydrochlorid, Salbutamol, 5-Fluoruracil und als Sexualhormon das Oestradiol sowie Gestagene wie Norethindronacetat, Levonorgestrel.

### Beispiel 1

Es wird zunächst eine Haftklebermasse HS hergestellt durch Homogenisieren von
a) 933 g eines Handelsproduktes (®Durotak 387-2516 der Fa. National Starch and Chemical, Zutphen, Niederlande - das ist eine 40 %ige Lösung eines selbstvernetzenden Acrylatpolymeren auf Basis von 2-Ethylhexylacrylat, Vinylacetat, Acrylsäure und Titanchelatester in einem Lösungsmittelgemisch aus Essigsäureethylester, Ethanol, Heptan und Methanol) mit
b) 8 g eines Triglycerids fraktionierter Kokosfettsäuren (C₈-C₁₀; ®Miglyol 812 der Fa. Hüls AG, Witten, Deutschland).

Daneben werden 6210 g ®Durotak 387-2516, 553 g Essigsäureethylester und 311 g Ethanol mit 66 g des zuvor genannten Triglyerids sowie 626 g eines Acrylharzes aus Dimethylaminoethylmethacrylat und neutralen Methacrylsäureestern (®Eudragit E 100 der Fa. Röhm-Pharma, Darmstadt, Deutschland) versetzt und homogenisiert (Klebermasse MS).

Daneben werden 72 g ®Eudragit E 100 in 101 g Nikotin eingetragen und darin gelöst. Es resultiert die Wirkstoffzubereitung.

Die Haftklebermasse HS wird so auf eine abhäsiv ausgerüstete Schutzschicht (A) aufgetragen, daß nach Abdampfen der Lösemittel eine Haftkleberschicht mit einem Flächengewicht von 40 g/m² gebildet wird.

Die Klebermasse MS wird so auf eine andere abhäsiv ausgerüstete Schutzschicht (B) aufgetragen, daß nach Abdampfen der Lösemittel ein Film mit einem Flächengewicht von 220 g/m² entsteht. Dieser Film wird auf die auf der Schutzschicht (A) aufgebrachte Haftkleberschicht aufkaschiert. Es resultiert die Unterbahn.

In einem weiteren Beschichtungsgang wird die Klebermasse MS so auf eine weitere abhäsiv ausgerüstete Schutzschicht (C) aufgetragen, daß nach Abdampfen der Lösemittel ein Film mit einem Flächengewicht von 110 g/m² entsteht, auf den die für den Wirkstoff undurchlässige Rückschicht aufkaschiert wird. Es wird dabei die Oberbahn gebildet.

Nach dem Abziehen der abhäsiv ausgerüsteten Schutzschicht (B) von der Unterbahn werden Ronden aus einem Vliesstoff (Fasergemisch Zellwolle/ Baumwolle 70:30 - Flächengewicht 40 g/m²) bzw. Papier (26 bzw. 23 g/m²) mittig positioniert.

Anschließend wird die Wirkstoffzubereitung auf die Vliesstoff- bzw. die Papierronden dosiert.

Die Oberbahn wird nach Abziehen der abhäsiv ausgerüsteten Schutzschicht (C) auf die Unterbahn (ausgerüstet mit Vliesstoff- bzw. Papierronden und dotiert mit Wirkstoffzubereitung) aufkaschiert, und es werden transdermale therapeutische Systeme ausgestanzt. Das Ergebnis ist in der Tabelle wiedergegeben.

| *Anzahl gefertigter* | *Reinigung des Tampons* | |
|---|---|---|
| *TTS* | *Vliesstoff* | *Papier* |
| 1.200 | Notwendig | nein |
| 2.400 | erneut notwendig | nein |
| 3.600 | erneut notwendig | nein |
| 4.800 | erneut notwendig | nein |
| über 100.000 | (fortlaufend nach jeweils 1.200 TTS) | nein |

Wie die Tabelle zeigt, können bei der Verwendung von Vliesstoff lediglich 1.200 transdermale therapeutische Systeme gefertigt werden. Danach ist eine Reinigung der Wirkstoffübertragungseinheit (Tampon) erforderlich. Dagegen lassen sich bei Einsatz von Papier mehr als 100.000 transdermale therapeutische Systeme fertigen, ohne daß es zu einem Maschinenstillstand aufgrund einer erforderlich werdenden Reinigung kommen muß.

### Beispiel 2

Entsprechend Beispiel 1 wurden transdermale therapeutische Systeme hergestellt und die Dosiergenauigkeit bestimmt.

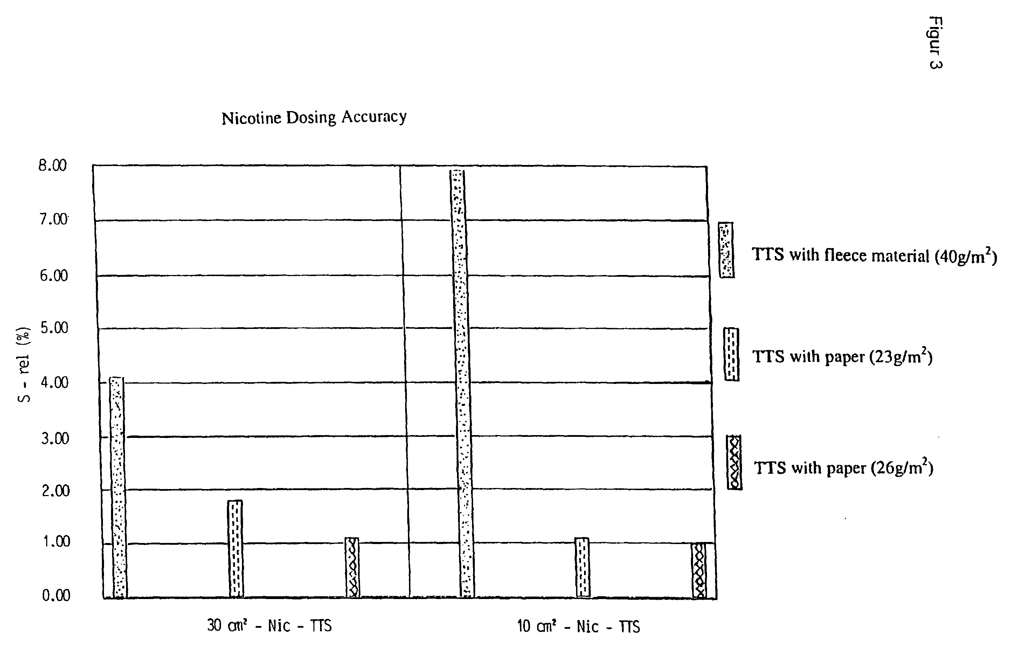

Es wurde bestimmt, wieviel Nikotin die einzelnen transdermalen therapeutischen Systeme enthielten und die Ergebnisse statistisch ausgewertet. Dabei zeigte sich, daß transdermale therapeutische Systeme, die unter Verwendung von Papier hergestellt wurden, eine deutlich geringere relative Standardabweichung (S-rel(%)) aufwiesen (siehe Figur 3).

## Patentansprüche

1. Transdermales therapeutisches System, enthaltend als wesentliche Merkmale
a) eine der Haut abgewandte, für den Wirkstoff undurchlässige Ruckschicht (10),
b) mindestens ein Wirkstoffdepot (14),
c) eine Matrix, die mit dem Wirkstoffdepot in Verbindung steht und die Abgabe des Wirkstoffs steuert (12), und
d) eine haftklebende Fixierungseinrichtung (16) für das therapeutische System auf der Haut (18), wobei das Depot und/oder die Matrix noch Stützmaterialien enthalten, **dadurch gekennzeichnet, daß** das Stützmaterial aus Papier besteht und der Wirkstoff Lidocain, Diphenylhydraminhydrochlorid, Salbutamol, 5-Fluoruracil, ein oder mehrere Sexualhormone, ein Gestagen oder Fentanyl ist.

2. Transdermales therapeutisches System nach Anspruch 1, **dadurch gekennzeichnet, daß** der Wirkstoff Östradiol, Norethindronacetat oder Levonorgestrel ist.

3. Transdermales therapeutisches System nach Anspruch 1 oder 2, **dadurch gekennzeichnet, daß** das Papier ein Flächengewicht von 9-60, vorzugsweise 15-40, insbesondere 20-35 g/m² hat.

4. Verfahren zur verbesserten Herstellung von transdermalen therapeutischen Systemen mit einer Streuung der aufgetragenen Wirkstoffmenge unter 2 %, **dadurch gekennzeichnet, daß** der Wirkstoff in üblicher Weise mittels eines Tampons auf ein Stützmaterial aufgetragen wird, das aus Papier besteht.

5. Verfahren nach Anspruch 4, **dadurch gekennzeichnet, daß** die Streuung der aufgetragenen Wirkstoffmenge unter 1,2 % liegt.

6. Verfahren nach Anspruch 4 oder 5, **dadurch gekennzeichnet, daß** das Papier ein Flächengewicht von 9-60, vorzugsweise 15-40, insbesondere 20-35 g/m² hat.

## Claims

1. A transdermal therapeutic system containing as essential features
a) a backing layer (10) remote from the skin and impermeable for the active substance,
b) at least one active substance depot (14),
c) a matrix containing the active substance depot and controlling the delivery of the active substance (12), and
d) a pressure-sensitive adhesive fixing device (16) for the therapeutic system on the skin (18), the depot or the matrix or both containing support materials, wherein the support material consists of paper and the acive substance is lidocaine, diphenylhydramine hydrochloride, salbutamol, 5-fluorouracil, one or more sexual hormones, a gestagen, or fentanyl.

2. The transdermal therapeutic system as claimed in claim 1, wherein the active substance is estradiol, norethindrone acetate or levonorgestrel.

3. The transdermal therapeutic system as claimed in claim 1 or 2, wherein the paper has a basis weight of from 9 to 60, preferably from 15 to 40 and particularly from 20 to 35 g/m².

4. A process for the improved production of a transdermal therapeutic system with a deviation of the amount of active substance applied of less than 2%, wherein the active substance is applied in conventional manner by means of a tampon to a support material which consists of paper.

5. The process as claimed in claim 4, wherein the deviation of the amount of active substance applied is less than 1.2%.

6. The process as claimed in claim 4 or 5, wherein the paper has a basis weight of from 9 to 60, preferably from 15 to 40 and particularly from 20 to 35 g/m².

## Revendications

1. Système thérapeutique transdermique, contenant comme caractéristiques essentielles
a) une couche dorsale (10) tournée vers la peau, imperméable pour la substance active,
b) au moins un dépôt de substance active (14),
d) une matrice, qui reste en relation avec le dépôt de substance active et régule l'administration de la substance active (12), et
e) un dispositif de fixation (16) adhérant par collage pour le système thérapeutique sur la peau (18), le dépôt et/ou la matrice contenant encore des matériaux protecteurs, **caractérisé en ce que** le matériau protecteur est constitué de papier et la substance active de lidocaine, de chlorhydrate de diphénylhydramine, de salbutamol, de 5-fluoruracile, d'une ou de plusieurs hormones sexuelles, d'une hormone sexuelle femelle ou de fentanyl.

2. Système thérapeutique transdermique selon la revendication 1, **caractérisé en ce que** la substance active est l'oestradiol, le noréthindron-acétate ou le lévonorgestrel.

3. Système thérapeutique transdermique selon la revendication 1 ou 2, **caractérisé en ce que** le papier a un grammage de 9-60, de préférence de 15-40, en particulier de 20-35 g/m².

4. Procédé pour améliorer la préparation de systèmes thérapeutiques transdermiques avec une dispersion de la quantité de substance active appliquée inférieure à 2 %, **caractérisé en ce que** la substance active est appliquée usuellement au moyen d'un tampon sur un matériau protecteur, qui est constitué de papier.

5. Procédé selon la revendication 4, **caractérisé en ce que** la dispersion de la quantité de substance active appliquée se situe à moins de 1,2 %.

6. Procédé selon la revendication 4 ou 5, **caractérisé en ce que** le papier a un grammage de 9-60, de préférence 15-40, en particulier 20-35 g/m².
